# NOUVEAU FASCICULE DE BREVET EUROPEEN

(11) **EP 0 655 244 B2**
(45) Date de publication et mention de la décision concernant l'opposition: **11.06.2008**
(45) Mention de la délivrance du brevet: 17.04.2002
(21) Numéro de dépôt: 94402420.7
(22) Date de dépôt: 27.10.1994
(51) Int. Cl.: A61K 31/198, A61P 3/00

(54) **Composition à base d'acides aminés pour le traitement d'infections**
Zusammensetzungen aus Aminosäure zur Behandlung von Infektionen
Amino acid composition for the treatment of infections

(30) Priorité: 28.10.1993 FR 9312884
(43) Date de publication de la demande: 31.05.1995
(73) Titulaire: SOCIETE DES PRODUITS NESTLE S.A., 1800 Vevey (CH); INSTITUT NATIONAL DE LA RECHERCHE AGRONOMIQUE (INRA), 75338 Paris Cédex 07 (FR)
(72) Inventeur: Arnal, Maurice, F-63540 Romagnat (FR); Rose, Francis, F-75003 Paris, France (FR); Breuille, Denis, F-63450 Saint-Saturnin, France (FR); Obled, Christiane, F-63450 Saint-Amant Tallende, France (FR)
(74) Mandataire: Casalonga, Axel

(56) Documents cités:
- EP-A- 0 046 167
- EP-A- 0 264 953
- EP-A- 0 318 446
- EP-A- 0 505 313
- EP-A- 0 567 216
- EP-A- 0 624 368
- DE-A- 2 946 563
- FR-A- 2 317 917
- US-A- 4 604 286
- NUTRITION (UNITED STATES), MAY-JUN 1991, VOL. 7, NO. 3, PAGE(S) 163-7;DISCUSSION 167-8, Garcia de Lorenzo y Mateos A et al 'Nutritional and metabolic support: converging concepts.'
- Article "Nutritional immunomodulation in burn patients" by J. Wesley Alexander, MD, ScD et al., Critical Care Medical, 1990, The Williams & Wilkins & Co., vol. 18, no. 2
- Article "Intact protein versus free amino acids in the nutritional support of thermally injured animals", O. Trock, M.S., R.D. et al., 1986, Journal of parenteral and enteral nutrition, vol. 10, no. 2
- Grimble et al., J. Nutr., vol. 122 (1992), pp. 2066-73

## Description

La présente invention a pour objet des compositions à base d'acides aminés présentées sous forme de solutés ou des préparations nutritives destinées à prévenir et/ou réduire les dommages tissulaires engendrés par les multiples dysfonctionnements métaboliques qui apparaissent, notamment à la suite d'une infection dite "sepsis".

L'infection peut être indépendante de toute autre pathologie, mais elle survient plus couramment chez l'homme après une intervention chirurgicale ou est associée à un traumatisme, à une brûlure, au diabète, à une cirrhose, à un néoplasme, etc... Elle peut également survenir lors d'un traitement avec des agents immunosuppresseurs, cytolytiques ou cytostatiques. Les accidents septiques sont également fortement corrélés avec un état de malnutrition, tout spécialement chez les jeunes enfants et chez les personnes âgées. De tels accidents existent également chez l'animal, notamment les animaux domestiques et notamment dans les élevages industriels (porcs, poussins, etc...).

La réponse métabolique à l'infection est complexe et, à ce jour, de très nombreux points restent encore non élucidés. Cette complexicité vient surtout du fait de la participation de nombreux facteurs : modification des apports des substrats aux différents organes et de leur utilisation, variation de la sensibilité et de la réactivité des tissus aux hormones, par exemple résistance à l'insuline, changement des débits sanguins, participation de nombreux médiateurs tels que le PAF, les cytokines (les interleukines, le TNF, etc...), dont les effets pharmacologiques peuvent être opposés selon le tissu considéré.

Cette réponse à l'infection est dynamique, avec plusieurs phases dont l'intensité et la durée dépendent de la sévérité de l'agression et du moment où survient l'infection par rapport à l'agression. Trois périodes sont habituellement discernées (CUTHBERTSON 1942). La phase initiale dite "ebb phase", pendant les 24 heures après l'agression qui se caractérise par une mobilisation rapide des substrats énergétiques et une activité métabolique diminuée, étape qui est suivie par la phase tardive dite "flow phase" dont la durée varie de quelques jours à 2 ou 3 semaines. Cette période voit l'activité métabolique s'accroître avec pour résultat un catabolisme général des tissus, en particulier du muscle. La dernière phase, chez les survivants, correspond à la convalescence qui est anabolique.

L'invention vise plus particulièrement à traiter ou prévenir par des compositions nutritionnelles les dysfonctionnements intervenant dans les deux premières phases qui sont caractérisées par l'existence d'une anorexie et d'une réponse hypermétabolique se traduisant cliniquement par une perte de poids et spécialement une fonte des protéines musculaires, par un état inflammatoire, l'existence d'une tachycardie, d'une hyperventilation, d'une consommation accrue d'oxygène, d'un dysfonctionnement du système immun, etc...

La déperdition accélérée des protéines du muscle sert à couvrir :
- les besoins augmentés en glucose de l'organisme par le biais de la néoglucogénèse hépatique et en glutamine, source énergétique essentielle pour les cellules de la muqueuse intestinale ou pour les cellules à multiplication rapide du système immun,
- les besoins d'acides aminés pour les synthèses protéiques augmentées de plusieurs organes, en particulier des protéines inflammatoires au niveau du foie.

Si les actions des hormones et de certains médiateurs, tels que le TNFα, ont été l'objet et font encore l'objet de nombreuses évaluations et que certains mécanismes commencent à être bien élucidés, les besoins nutritionnels spécifiques, plus particulièrement ceux qui concernent les acides aminés, restent encore très mal connus dans le cas d'une infection ou des réactions inflammatoires post-agressions.

La demanderesse a eu l'idée que la stimulation subite des synthèses d'un grand nombre de protéines inflammatoires et de défense, vitales pour l'organisme, faiblement sécrétées en situation physiologique normale, riches en certains acides aminés, nécessitait en particulier, dans le cas d'une infection, des apports supplémenaires. Lors de cette phase aiguë, vu l'état d'anorexie des patients ou des animaux, la synthèse de ces protéines implique que l'organisme dégrade des quantités importantes de protéines musculaires ou autres, pour disposer d'une quantité suffisante en ces acides aminés. La consommation accrue de glutamine, comme source énergétique par le tractus digestif au cours des diverses agressions, en est une illustration.

Les protéines hépatiques de l'inflammation, telles que la C-réactive-protéine, l'α₁-1-antichymotrypsine, l'α₁-acide glycoprotéine, le fibrinogène, l'haptoglobuline, l'α₂-macroglobuline (chez le rat), les métallothionéines contiennent un pourcentage élevé de cystéine, de sérine, d'acide aspartique, d'asparagine, de thréonine comparé à celles des protéines du muscle. La thréonine, l'acide aspartique et l'asparagine sont des acides aminés sur lesquels sont liés les oses constitutifs de ces divers glycoprotéines.

Lors des réactions de défense, du fait de l'état d'anorexie, l'organisme, pour synthétiser ces diverses protéines vitales, doit dégrader ses protéines musculaires de façon significative pour couvrir ces besoins particuliers, d'autant qu'à l'inverse des protéines myofibrillaires, les protéines de la réaction inflammatoire ont, en général, des demi-vies brèves.

La couverture des besoins en acides aminés doit, non seulement permettre à l'organisme d'améliorer les synthèses des protéines vitales, mais aussi d'éviter la perte des protéines musculaires. L'organisme ne possédant pas de protéines de réserve, toute quantité de protéines perdue correspond à une perte de fonction. La diminution des protéines musculaires entraîne une altération des capacités respiratoires du malade, mais aussi de ses capacités motrices. Il s'ensuit une période de convalescence longue, étant donné que la régénération des protéines myofibrillaires est lente. Cette couverture des besoins entraîne un temps de maladie plus court et permet de raccourcir la durée d'hospitalisation. Elle permet également un meilleur rendement de protection des animaux domestiques.

Il a également été mis en évidence que, durant cette période de choc, avec l'existence d'un hypermétabolisme persistant, il existait une production abondante de radicaux libres dont les effets nocifs ont été largement décrits. Pour lutter contre ces processus oxydatifs, l'organisme dispose de substances antioxydantes et de "pièges à radicaux". Le glutathion, tripeptide composé de glycine, d'acide glutamique et de cystéine, est l'un des plus abondants. Le besoin accru de ce dérivé implique, pour l'organisme, de disposer en quantité suffisante pour sa synthèse, des trois acides aminés qui le constituent.

La déplétion en glutathion, au niveau cellulaire, a pour l'organisme des conséquences métaboliques délétères : en plus de son rôle comme capteur de radicaux libres, il intervient dans de multiples réactions du métabolisme (coenzyme de réactions enzymatiques, synthèse des déoxyribonucléotides, métabolisme des xénobiotiques, réducteur intracellulaire) et il est lui-même une réserve de cystéine, directement disponible pour la synthèse protéique.

La demanderesse a découvert, après mise au point d'un modèle d'infection chez le rat, consistant en une injection unique de bactéries vivantes (E.Coli) qui maintient les animaux dans une situation catabolique durant plusieurs jours, que le besoin en certains acides aminés était augmenté.

Elle a pu constater qu'au cours d'une infection induite, on observe chez les animaux infectés, comparés à des animaux nourris de façon appariée, un amaigrissement important pendant 2 à 3 jours, avec l'instauration d'une anorexie sévère, un taux de TNF α circulant élevé supérieur à 10 ng/ml, une teneur plasmatique d'α 1 glycoprotéine acide multipliée par 20 à 60, une hyperglycémie (1,82 g/l), une hyperinsulinémie (34,7 µu/ml).

Les mesures de synthèse protéiques, appréciées par la technique des larges doses, ont montré, toujours par rapport à des animaux nourris de façon appariée, que dans le foie, la vitesse de synthèse était augmentée de 1,8 à 2,7 fois, tandis que dans le muscle, elle était diminuée de 30%. Dans ce dernier tissu, on observe un accroissement de la protéolyse.

La synthèse protéique du corps entier, moins celle du foie, est augmentée malgré une forte diminution des synthèses du muscle. Cela suppose que, dans d'autres organes, les synthèses sont stimulées. n a été possible, en particulier, de constater une augmentation de la synthèse protéique dans la rate et le poumon.

L'étude des bilans de fixation et d'oxydation des acides aminés au cours de ce modèle d'infection a permis, effectivement, de déterminer un besoin accru de plusieurs acides aminés essentiels et non essentiels, et plus particulièrement au niveau du foie. Le contenu protéique du foie des rats infectés, augmente de 42% comparé à des témoins pair-fed. La demanderesse a pu constater plus particulièrement l'augmentation de la concentration en cystéine de l'ordre de 74%.

L'analyse du contenu en acides aminés du corps entier a montré une forte diminution chez les animaux infectés, sauf pour l'ensemble cystéine-cystine qui augmente significativement de 9% par rapport aux témoins nourris de façon appariée et pour certains acides aminés (thréonine, arginine) qui se maintiennent au même niveau. Ceci indique une épargne de ces acides aminés, puisque les rats infectés catabolisent respectivement 3,7 et 54% moins de thréonine et de cystéine que les témoins nourris de façon appariée, contrairement à tous les autres acides aminés indispensables qui montrent des oxydations augmentées de 10 à 30% lors de l'infection.

L'analyse de la répartition de la radioactivité dans les différents tissus après injection de L³⁵S cystéine à des rats sur le modèle d'infection précédemment cité, a fait apparaître une utilisation accrue de la cystéine pour synthétiser les protéines de la réaction inflammatoire et le glutathion. En effet, la radioactivité incorporée par gramme de protéines dans la rate et dans les protéines plasmatiques moins l'albumine, augmente de 70% chez les animaux infectés par rapport à leurs témoins pair-fed. Le pourcentage de la dose injectée se trouvant dans une fraction contenant principalement la cystéine et le glutathion, est respectivement 1,9 et 4 fois plus élevé dans le foie et la rate d'animaux infectés comparé à des témoins pair-fed.

L'effet de la supplémentation en acides aminés des régimes sur les pertes de poids a confirmé le besoin accru en certains acides aminés lors d'une infection. Trois lots d'animaux recevant des régimes isoazotés ont été comparés. Un lot témoin (lot T), un lot recevant un régime supplémenté en thréonine, sérine, acide aspartique, asparagine et arginine (lot AA) et un lot recevant un régime supplémenté en thréonine, sérine, acide aspartique, asparagine, arginine et cystéine (lot Cys). Ces supplémentations ont permis de limiter la perte de poids et d'accélérer la reprise de croissance des animaux infectés. Dix jours après l'infection, le poids des animaux était 14%, 8% et 3,5% plus faible que leur poids initial dans les lots T, AA et Cys respectivement. L'augmentation de la teneur en cystéine du régime de 0,8% à 6,7% a entraîné une réduction de l'excrétion azotée des animaux infectés par rapport à leurs témoins pair-fed d'environ 35% dans les jours qui suivent l'infection. Dans cette même étude, la supplémentation du régime en cystéine a permis de normaliser la concentration en glutathion du foie, celle-ci étant diminuée de l'ordre de 25% avec le régime ne contenant que 0,8% de cystéine.

Ces résultats ont permis à la demanderesse d'établir qu'au cours de l'infection en particulier et plus généralement lors du déclenchement de situations hautement cataboliques et hypermétaboliques, les besoins en cystéine et à moindre échelle en thréonine, en sérine, en acide aspartique et asparagine, sont nettement augmentés.

L'invention réside dans la formulation de compositions d'acides aminés présents dans des proportions telles qu'elles couvrent les besoins spécifiques en acides aminés et permettent d'éviter ou de prévenir la perte d'une masse importante de protéines musculaires.

Un objet de l'invention est donc constitué par une composition d'acides aminés destinée à être administrée par voie orale, par voie entérale ou parentérale, permettant de résoudre ce problème.

L'invention a également pour objet l'utilisation d'une composition d'acides aminés particulière, pour la prépration d'un médicament destiné à traiter les dommages tissulaires engendrés par des dysfonctionnements métaboliques apparaissant en particulier à la suite d'une infection.

D'autres objets de l'invention apparaîtront à la lecture de la description et des exemples qui suivent.

La composition à base d'acides aminés destinée à être administrée par voie orale, entérale ou parentérale, conforme à l'invention est caractérisée par le fait qu'elle contient dans un milieu biologiquement et nutritionnellement acceptable, au moins de la cystéine libre ou sous forme de précurseur, prodrogue, protéine, hydrolysat peptidique, dans une proportion égale ou supérieure à 3% par rapport à la totalité des acides aminés présents dans la composition ; au moins de la thréonine dans des proportions égales ou supérieures à 5% et au moins de la sérine dans des proportions égales ou supérieures à 12% et au moins de l'acide aspartique ou de l'asparagine dans des proportions supérieures ou égales à 10%, ces proportions étant déterminées par rapport à la quantité d'acides aminés présents dans la composition ; et des sources de calories osidiques et lipidiques.

Un mode de réalisation est constitué par une composition sous forme d'une composition nutritive destinée à la voie orale ou entérale, caractérisée par le fait qu'elle contient dans un milieu biologiquement et nutritionnellement acceptable, au moins de la cystéine libre ou sous forme de leurs sels, protéine ou hydrolysat peptidique, dans des proportions égales ou supérieures à 3% par rapport à la totalité des acides aminés présents dans la composition, et par le fait qu'elle renferme en plus des sources caloriques osidiques et/ou lipidiques, des électrolytes, des oligo-éléments et/ou des vitamines et au moins de là thréonine dans des proportions égales ou supérieures à 5% et au moins de la serine dans des proportions égales ou supérieures à 12% et au moins de l'acide aspartique ou de l'asparagine dans des proportions égales ou supérieures à 10%, ces proportions étant déterminées par rapport à la totalité des acides aminés présents dans la composition.

Une forme de réalisation particulièrement préférée consiste à utiliser des compositions telles que définies précédemment et contenant les 8 acides aminés essentiels, à savoir la leucine, l'isoleucine, la valine, le tryptophane, la phénylalanine, la lysine, la méthionine, la thréonine.

Selon une autre forme de réalisation de l'invention, la composition contient également de la glycine et/ou de l'arginine.

La composition conforme à l'invention peut également contenir de la taurine et/ou de la glutamine.

Les compositions conformes à l'invention se présentent notamment sous forme de soluté qui est un mélange d'acides aminés éventuellement utilisés sous forme de leurs sels pharmaceutiquement acceptables dans un milieu constitué généralement par de l'eau distillée.

Les compositions conformes à l'invention peuvent contenir en particulier pour 1 litre de solution d'acides aminés, les constituants suivants dans les quantités suivantes :

| | |
|---|---|
| Leucine | 5 à 12 g/l |
| Isoleucine | 3 à 10 g/l |
| Valine | 5 à 10 g/l |
| Tryptophane | 1,0 à 3 g/l |
| Phénylalanine | 1,5 à 7 g/l |
| Lysine | 2 à 7 g/l |
| Méthionine | 1,5 à 5 g/l |
| Thréonine | 3,0 à 7 g/l |

Cette composition peut éventuellement contenir de la sérine dans des proportions de 2,5 à 6 g/l, de l'acide aspartique dans des proportions de 1,5 à 4 g/l, de la glycine dans des proportions comprises entre 3 à 7 g/l, de l'arginine dans des proportions comprises entre 5 et 10 g/l, de la taurine dans des proportions comprises entre 1 et 4 g/l, de la glutamine dans des proportions supérieures ou égales à 4 g/l.

L'invention se caractérise plus particulièrement par le fait que la cystéine est présente dans cette composition dans des proportions comprise de préférence entre 3 et 10%.

La thréonine, comme déjà indiqué ci-dessus, est présente dans des proportions comprises de préférence entre 5 et 12% par rapport à la quantité totale d'acides aminés présents.

La sérine est présente dans des proportions de préférence comprises entre 12 et 16% par rapport au poids total des acides aminés présents. L'acide aspartique ou l'asparagine le sont de préférence dans des proportions comprises de préférence entre 10 et 15% par rapport au poids total des acides aminés présents.

La cystéine, utilisée conformément à l'invention, peut être utilisée sous forme de prodrogue ou de sel pharmaceutiquement acceptable, tel que sous forme de l'acide L-oxothiazolidine carboxylique. Notamment lorsqu'on souhaite éviter de maintenir des taux plasmatiques élevés de cystéine. Il est bien entendu qu'on peut utiliser d'autres précurseurs ou dérivés de cystéine pouvant être convertis en cystéine à l'intérieur des cellules. La cystéine peut être mise en oeuvre sous forme combinée avec d'autres acides aminés tels que sous forme de protéine, peptide.

Les quantités en prodrogue ou précurseurs de cystéine, peptide ou protéine, sont déterminées sur la base de la cystéine susceptible d'être libérée à partir de ces dérivés.

On peut également utiliser les autres acides aminés mentionnés ci-dessus sous forme de précurseurs ou de prodrogues, tels que par exemple sous forme de dipeptides, notamment dans le cas de l'acide aspartique et/ou de l'asparagine.

Les compositions conformes à l'invention peuvent se présenter non seulement sous forme de solution aqueuse, mais également sous d'autres formes. C'est ainsi que la cystéine peut être administrée en modifiant simplement des formules orales entérales existantes en y introduisant la quantité de cystéine compatible avec les proportions conformes à l'invention.

La supplémentation de cystéine peut également se faire dans des préparations destinées à la nutrition orale ou entérale. Elle peut être effectuée dans ce cas par l'utilisation de protéines ou d'hydrolysats peptidiques naturellement riches en cystéine/cystine.

Le taux de cystéine doit dans ce cas également être présent dans des quantités supérieures à la proportion de cystéine présente dans une composition destinée à l'homme sain. Cette quantité étant déterminée par rapport à la totalité des acides aminés présents sous forme libre ou combinée. Il est également possible de l'exprimer en tenant compte de la teneur en azote contenu dans la cystéine ou de ces précurseurs et celle de la quantité totale d'azote dans la composition. Le pourcentage représente dans ce cas la quantité d'azote de la cystéine par rapport à l'azote total.

La cystéine liée dans une protéine ou un hydrolysat peptidique, est présente dans des proportions égales ou supérieures à 3% par rapport à la totalité des acides aminés présents sous forme libre ou liée dans la composition.

Lorsqu'elle est exprimée en teneur en azote, la quantité d'azote de la cystéine libre ou sous forme de l'un de ses précurseurs, prodrogue, protéine, hydrolysat peptidique, est supérieure ou égale à 2,15% par rapport à l'azote total.

Ces compositions peuvent se présenter sous forme d'une composition nutritive complète destinée à l'administration par voie parentérale. Cette préparation renferme, outre les acides aminés ou leurs dérivés (peptides), et des sources de calories osidiques (glucose, fructose, sorbitol, etc.) et/ou lipidiques (triglycérides d'acides gras à chaînes longues ou à chaînes moyennes ou courtes), des électrolytes, des oligo-éléments, et des vitamines. La cystéine ou ses précurseurs étant présents dans des proportions supérieures à 3% par rapport à la quantité d'acides aminés présents dans la composition nutritive, de même que la thréonine, la sérine, l'acide aspartique ou asparagine dans les proportions précitées.

La composition se présente sous forme d'une composition nutritive destinée à la voie orale ou entérale contenant de la cystéine dans des proportions supérieures à 3% par rapport à la quantité d'acides aminés présents dans la composition nutritive ainsi que la thréonine, la sérine, l'acide aspartique ou l'asparagine dans les proportions précitées. L'ajout en cystéine ou par les autres acides aminés cités ci-dessus, est obtenue soit par l'acide aminé lui-même, par une prodrogue ou par des protéines ou des hydrolysats peptidiques particulièrement riches en l'acide aminé considéré (par exemple cystéine). Cette composition, outre des protéines, des acides aminés et des peptides, renferme des sources caloriques osidiques (sous forme de carbohydrates divers) et/ou lipidiques (triglycérides d'acides gras à chaînes longues ou moyennes, apportés sous forme d'huiles de diverses origines), des électrolytes, des oligo-éléments et des vitamines.

La cystéine peut également être prémélangée avec les autres acides aminés utilisables dans les compositions conformes à l'invention et se présenter sous forme de poudre aseptique réhydratable au moment de l'administration ou pouvant être stockée sous forme d'un concentrat congelé ou réfrigéré qui est décongelé et mélangé à la concentration appropriée au moment de l'utilisation.

Les compositions parentérales peuvent se présenter sous forme de solution aqueuse ou de solution non aqueuse, de suspension ou d'émulsion.

Ces compositions peuvent être administrées par des dispositifs connus dans les modes d'administration orale, parentérale ou entérale.

Un autre objet de l'invention est constitué par l'utilisation d'une composition telle que définie ci-dessus, pour la préparation d'un médicament destiné à prévenir ou à diminuer les dommages tissulaires engendrés par des dysfonctionnements métaboliques. Le traitement s'effectue comme indiqué ci-dessus par voie parentérale, entérale ou orale.

Le traitement de dommages tissulaires engendrés par les dysfonctionnements métaboliques apparaissant en particulier à la suite d'une infection, est fait en administrant par voie parentérale ou entérale à l'homme ou à l'animal une composition telle que définie ci-dessus.

Les exemples suivants sont destinés à illustrer l'invention sans présenter un caractère limitatif.

### EXEMPLES

Exemples 1 et 2 - On prépare les solutés d'acides aminés suivants :

| | | |
|---|---|---|
| Leu | 7,2 g/l | 7,2 g/l |
| Ile | 5,6 g/l | 5,6 g/l |
| Val | 5,6 g/l | 5,6 g/l |
| Trp | 1,2 g/l | 1,2 g/l |
| Phe | 3,2 g/l | 3,2 g/l |
| Lys | 3,2 g:l | 3,2 g/l |
| Met | 2 g/l | 2 g/l |
| Thr | 4 g/l | 6 g/l |
| | | |
| Asp | 8 g/l | 8,5 g/l |
| Glu | 2,4 g/l | 2,4 g/l |
| Ser | 9,8 g/l | 9,8 g/l |
| Gly | 5,9 g/l | 5 g/l |
| Ala | 6,2 g/l | 5,8 g/l |
| Cys | 2,5 g/l | 4 g/l |
| Orn | 2,4 g/l | - |
| Tyr | 0,4 g/l | 0,4 g/l |
| His | 3,2 g/l | 3 g/l |
| Arg | 4,6 g/l | 4,6 g/l |
| Pro | 3,2 g/l | 3,2 g/l |
| | | |
| Eau distillée qsp | 1 l | |
| | | |
| AAT | 80,2 g/l | 80,7 g/l |

Exemple 3 - On prépare les solutés d'acides aminés suivants :

| | |
|---|---|
| Leu | 6 g/l |
| Ile | 4,5 g/l |
| Val | 4,5 g/l |
| Trp | 1,2 g/l |
| Phe | 3 g/l |
| Lys | 3 g/l |
| Met | 2 g/l |
| Thr | 6 g/l |
| | |
| Asp | 8,5 g/l |
| Gln | 8 g/l |
| Ser | 9,8 g/l |
| Gly | 4,8 g/l |
| Ala | 4 g/l |
| Cys | 4 g/l |
| Orn | - |
| Tyr | 0,4 g/l |
| His | 3 g/l |
| Arg | 4 g/l |
| Pro | 3 g/l |
| | |
| Eau qsp | 1 l |
| | |
| AAT | 81,1 g/l |

Exemple 4 - On prépare les solutés d'acides aminés suivants :

| | |
|---|---|
| Leu | 12 g/l |
| Ile | 9,3 g/l |
| Val | 9,3 g/l |
| Trp | 2 g/l |
| Phe | 5,33 g/l |
| Lys | 5,33 g/l |
| Met | 3,33 g/l |
| Thr | 10 g/l |
| | |
| Asp | 14,16 g/l |
| Glu | 4 g/l |
| Ser | 16,8 g/l |
| Gly | 8,33 g/l |
| Ala | 10 g/l |
| Cys | 6,66 g/l |
| Orn | - |
| Tyr | 0,5 g/l |
| His | 5 g/l |
| Arg | 7,6 g/l |
| Pro | 5,18 g/l |
| | |
| Eau qsp | 1 l |
| | |
| AAT | 134,17 g/l |

Exemples 5 et 6 - On prépare les solutés d'acides aminés suivants :

| | | |
|---|---|---|
| Leu | 7,2 g/l | 7,2 g/l |
| Ile | 5,6 g/l | 5,6 g/l |
| Val | 5,6 g/l | 5,6 g/l |
| Trp | 1,2 g/l | 1,2 g/l |
| Phe | 3,2 g/l | 3,2 g/l |
| Lys | 3,2 g:l | 3,2 g/l |
| Met | 2 g/l | 2 g/l |
| Thr | 4 g/l | 6 g/l |
| | | |
| Asp | 8 g/l | 8,5 g/l |
| Glu | 2,4 g/l | 2,4 g/l |
| Ser | 9,8 g/l | 9,8 g/l |
| Gly | 5,6 g/l | 5 g/l |
| Ala | 6 g/l | 5,8 g/l |
| OTC° | 2,6 g/l | 4 g/l |
| Orn | 2,4 g/l | - |
| Tyr | 0,4 g/l | 0,4 g/l |
| His | 3,2 g/l | 3 g/l |
| Arg | 4,8 g/l | 4,6 g/l |
| Pro | 3,2 g/l | 3,2 g/l |
| | | |
| Eau qsp | 1 l | |
| | | |
| AAT | 80,4 g/l | 80,7 g/l |

| | | |
|---|---|---|
| ° Acide 4-oxothiazolidine-carboxylique ou sous forme salifiée. | | |

Exemple 7 - On prépare les solutés d'acides aminés suivants :

| | |
|---|---|
| Leu | 6 g/l |
| Ile | 4,5 g/l |
| Val | 4,5 g/l |
| Trp | 1,2 g/l |
| Phe | 3 g/l |
| Lys | 3 g/l |
| Met | 2 g/l |
| Thr | 6 g/l |
| | |
| Asp | 8,5 g/l |
| Gln | 7,8 g/l |
| Ser | 9,8 g/l |
| Gly | 5 g/l |
| Ala | 4 g/l |
| OTC° | 4 g/l |
| Orn | - |
| Tyr | 0,4 g/l |
| His | 3 g/l |
| Arg | 4 g/l |
| Pro | 3 g/l |
| | |
| Eau qsp | 1 l |
| | |
| AAT | 81,1 g/l |

| | |
|---|---|
| ° Acide 4-oxothiazolidine-carboxylique ou sous forme salifiée. | |

Exemple 8 - On prépare les solutés d'acides aminés suivants :

| | |
|---|---|
| Leu | 12 g/l |
| Ile | 9,3 g/l |
| Val | 9,3 g/l |
| Trp | 2 g/l |
| Phe | 5,33 g/l |
| Lys | 5,33 g/l |
| Met | 3,33 g/l |
| Thr | 10 g/l |
| | |
| Asp | 14,16 g/l |
| Gln | 4 g/l |
| Ser | 16 g/l |
| Gly | 8,33 g/l |
| Ala | 10 g/l |
| OTC° | 6,66 g/l |
| Orn | - |
| Tyr | 0,5 g/l |
| His | 5 g/l |
| Arg | 7,6 g/l |
| Pro | 5,33 g/l |
| | |
| Eau qsp | 1 l |
| | |
| AAT | 134,17 g/l |

| | |
|---|---|
| ° Acide 4-oxothiazolidine-carboxylique ou sous forme salifiée. | |

Exemple 9 - On prépare les solutés d'acides aminés suivants :

| | |
|---|---|
| Leu | 6 g/l |
| Ile | 5 g/l |
| Val | 5 g/l |
| Trp | 1,2 g/l |
| Phe | 3 g/l |
| Lys | 3 g/l |
| Met | 2 g/l |
| Thr | 6 g/l |
| | |
| Asp | 8,5 g/l |
| Ser | 9,6 g/l |
| Cys | 5 g/l |
| Ala-Gln | 15 g/l |
| Gly | 5 g/l |
| Arg | 4 g/l |
| | |
| Eau qsp | 1 l |
| | |
| AAT | 78,3 g/l |

Exemple 10 - On prépare les solutés d'acides aminés suivants :

| | |
|---|---|
| Leu | 6 g/l |
| Ile | 5 g/l |
| Val | 5 g/l |
| Trp | 1,2 g/l |
| Phe | 3 g/l |
| Lys | 3 g/l |
| Met | 2 g/l |
| Thr | 6 g/l |
| | |
| Asp | 8,5 g/l |
| Ser | 9,6 g/l |
| OTC° | 5 g/l |
| Ala-Gln | 15 g/l |
| Gly | 5 g/l |
| Arg | 4 g/l |
| | |
| Eau qsp | 1 l |
| | |
| AAT | 78,3 g/l |

| | |
|---|---|
| °Acide 4-oxothiazolidine-carboxylique ou sous forme salifiée. | |

## Revendications

1. Composition à base d'acides aminés destinée à être administrée par voie orale, entérale ou parentérale, **caractérisée par le fait qu'**elle contient dans un milieu biologiquement et nutritionnellement acceptable, au moins de la cystéine libre ou sous forme de précurseur, prodrogue, protéine, hydrolysat peptidique, dans une proportion égale ou supérieure à 3% par rapport à la totalité des acides aminés présents dans la composition ; au moins de la thréonine dans des proportions égales ou supérieures à 5% et au moins de la sérine dans des proportions égales ou supérieures à 12% et au moins de l'acide aspartique ou de l'asparagine dans des proportions supérieures ou égales à 10%, ces proportions étant déterminées par rapport à la quantité d'acides aminés présents dans la composition ; et des sources de calories osidiques et lipidiques.

2. Composition selon la revendication 1, sous forme d'une composition nutritive complète destinée à l'administration par voie parentérale, cette préparation renferme, outre les acides aminés ou leurs dérivés (peptides), et les sources de calories osidiques et lipidiques, des électrolytes, des oligo-éléments, et/ou des vitamines.

3. Composition selon la revendication 1, **caractérisée par le fait que** la composition se présente sous forme d'une composition orale ou entérale nutritive.

4. Composition sous forme d'une composition nutritive destinée à la voie orale ou entérale, **caractérisée par le fait qu'**elle contient dans un milieu biologiquement et nutritionnellement acceptable, au moins de la cystéine libre ou sous forme de leurs sels, protéine ou hydrolysat peptidique, dans des proportions égales ou supérieures à 3% par rapport à la totalité des acides aminés présents dans la composition, et **par le fait qu'**elle renferme en plus des sources caloriques osidiques et/ou lipidiques, des électrolytes, des oligo-éléments et/ou des vitamines et au moins de la thréonine dans des proportions égales ou supérieures à 5% et au moins de la sérine dans des proportions égales ou supérieures à 12% et au moins de l'acide aspartique ou de l'asparagine dans des proportions égales ou supérieures à 10%, ces proportions étant déterminées par rapport à la totalité des acides aminés présents dans la composition.

5. Composition selon l'une quelconque des revendications 1 à 4, **caractérisée par le fait que** la quantité d'azote de la cystéine est supérieure ou égale à 2,15% par rapport à la quantité d'azote totale.

6. Composition selon l'une quelconque des revendications 1 à 5, **caractérisée par le fait qu'**elle contient au moins les 8 acides aminés essentiels constitués par la leucine, l'isoleucine, la valine, le tryptophane, la phénylalanine, la lysine, la méthionine, la thréonine.

7. Composition selon l'une quelconque des revendications 1 à 6, **caractérisée par le fait que** la composition contient également de la glycine et/ou de l'arginine.

8. Composition selon l'une quelconque des revendications 1 à 7, **caractérisée par le fait que** la composition contient également de la taurine et/ou de la glutamine.

9. Composition selon l'une quelconque des revendications 1 à 8, **caractérisée par le fait qu'**elle contient pour 1 litre de solution d'acides aminés :
| | |
|---|---|
| Leucine | 5 à 12 g/l |
| Isoleucine | 3 à 10 g/l |
| Valine | 5 à 10 g/l |
| Tryptophane | 1,0 à 3 g/l |
| Phénylalanine | 1,5.à 7 g/l |
| Lysine | 2 à 7 g/l |
| Méthionine | 1,5 à 5 g/l |
| Thréonine | 3,0 à 7 g/l |

10. Composition selon l'une quelconque des revendications 1 à 9, **caractérisée par le fait qu'**elle se présente sous forme d'une poudre réhydratable au moment de l'administration.

11. Utilisation, pour la préparation d'un médicament destiné à prévenir ou à diminuer les dommages tissulaires engendrés par des dysfonctionnements métaboliques, d'une composition à base d'acides aminés destinée à être administrée par voie orale, entérale ou parentérale, qui contient dans un milieu biologiquement et nutritionnellement acceptable, au moins de la cystéine libre ou sous forme de précurseur, prodrogue, protéine, hydrolysat peptidique, dans une proportion égale ou supérieure à 3% par rapport à la totalité des acides aminés présents dans la composition et au moins de la thréonine dans des proportions égales ou supérieures à 5% et au moins de la sérine dans des proportions égales ou supérieures à 12% et au moins de l'acide aspartique ou de l'asparagine dans des proportions égales ou supérieures à 10%, ces proportions étant déterminées par rapport à la totalité des acides aminés présents dans la composition.

12. Utilisation selon la revendication 11, **caractérisée par le fait que** les dysfonctionnements métaboliques sont dus à une infection ou à une agression engendrant une réaction inflammatoire.

13. Utilisation selon l'une quelconque des revendications 1 à 12, **caractérisée en ce que** la composition contient au moins les 8 acides aminés essentiels constitués par la leucine, l'isoleucine, la valine, le tryptophane, la phénylalanine, la lysine, la méthionine, la thréonine.

14. Utilisation selon l'une quelconque des revendications 1 à 13, **caractérisée par le fait que** la composition contient également de la glycine et/ou de l'arginine et /ou de la taurine et/ou de la glutamine.

15. Utilisation selon l'une quelconque des revendications 1 à 14, **caractérisée en ce que** la composition contient pour 1 litre de solution d'acides aminés :
| | |
|---|---|
| Leucine | 5 à 12 g/l |
| Isoleucine | 3 à 10 g/l |
| Valine | 5 à 10 g/l |
| Tryptophane | 1,0 à 3 g/l |
| Phénylalanine | 1,5 à 7 g/l |
| Lysine | 2 à 7 g/l |
| Méthionine | 1,5 à 5 g/l |
| Thréonine | 3,0 à 7 g/l |

16. Utilisation selon l'une quelconque des revendications 11 à 15, **caractérisée par le fait que** la composition est destinée à être administrée à l'homme.

17. Utilisation selon l'une quelconque des revendications 11 à 15, **caractérisée par le fait que** la composition est destinée à être administrée à l'animal.

## Claims

1. Composition based on amino acids intended to be administered orally, enterally or parenterally, **characterized in that** it contains, in a biologically and nutritionally acceptable medium, at least free cysteine or cysteine in the form of a precursor, prodrug, protein or peptide hydrolysate, in a proportion equal to or greater than 3% with respect to all the amino acids present in the composition; at least threonine in proportions equal to or greater than 5% and at least serine in proportions equal to or greater than 12% and at least aspartic acid or asparagine in proportions greater than or equal to 10%, these proportions being determined with respect to the amount of amino acids present in the composition; and carbohydrate and lipid calorie sources.

2. Composition according to Claim 1, in the form of a complete nutritive composition intended for parenteral administration, this preparation containing, besides the amino acids or their derivatives (peptides) and carbohydrate and lipid calorie sources, electrolytes, trace elements and/or vitamins.

3. Composition according to Claim 1, **characterized in that** the composition is provided in the form of a nutritive oral or enteral composition.

4. Composition in the form of a nutritive composition intended for the oral or enteral route, **characterized in that** it contains, in a biologically and nutritionally acceptable medium, at least free cysteine or cysteine in the form of its salts or of a protein or peptide hydrolysate, in proportions equal to or greater than 3% with respect to all the amino acids present in the composition, and **in that** it additionally contains carbohydrate and/or lipid calorie sources, electrolytes, trace elements and/or vitamins, and at least threonine in proportions equal to or greater than 5% and at, least serine in proportions equal to or greater, than 12% and at least aspartic acid or asparagine in proportions equal to or greater than 10%, these proportions being determined with respect to all the amino acids present in the composition.

5. Composition according to any one of Claims 1 to 4, **characterized in that** the amount of nitrogen from the cysteine is greater than or equal to 2.15% with respect to the total amount of nitrogen.

6. Composition according to any one of Claims 1 to 5, **characterized in that** it contains at least the 8 essential amino acids consisting of leucine, isoleucine, valine, tryptophan, phenylalanine, lysine, methionine and threonine.

7. Composition according to any one of Claims 1 to 6, **characterized in that** the composition also contains glycine and/or arginine.

8. Composition according to any one of Claims 1 to 7, **characterized in that** the composition also contains taurine and/or glutamine.

9. Composition according to any one of Claims 1 to 8, **characterized in that** it contains, per 1 litre of amino acids solution:
| | |
|---|---|
| Leucine | 5 to 12 g/l |
| Isoleucine | 3 to 10 g/l |
| Valine | 5 to 10 g/l |
| Tryptophan | 1.0 to 3 g/l |
| Phenylalanine | 1.5 to 7 g/l |
| Lysine | 2 to 7 g/l |
| Methionine | 1.5 to 5 g/l |
| Threonine | 3.0 to 7 g/l. |

10. Composition according to any one of Claims 1 to 9, **characterized in that** it is provided in the form of a powder which can be rehydrated at the time of administration.

11. Use, for the preparation of a medicament intended to prevent or decrease tissue damage brought about by metabolic dysfunctions, of a composition based on amino acids intended to be administered orally, enterally or parenterally, which contains, in a biologically and nutritionally acceptable medium, at least free cysteine or cysteine in the form of a precursor, prodrug, protein or peptide hydrolysate, in a proportion equal to or greater than 3% with respect to all the amino acids present in the composition, and at least threonine in proportions equal to or greater than 5% and at least serine in proportions equal to or greater than 12% and at least aspartic acid or asparagine in proportions equal to or greater than 10%, these proportions being determined with respect to all the amino acids present in the composition.

12. Use according to Claim 11, **characterized in that** the metabolic dysfunctions are due to an infection or to an attack bringing about an inflammatory reaction.

13. Use according to either one of Claims 11 and 12, **characterized in that** the composition contains at least the 8 essential amino acids consisting of leucine, isoleucine, valine, tryptophan, phenylalanine, lysine, methionine and threonine.

14. Use according to any one of Claims 11 to 13, **characterized in that** the composition also contains glycine and/or arginine and/or taurine and/or glutamine.

15. Use according to any one of Claims 11 to 14, **characterized in that** the composition contains, per 1 litre of amino acids solution:
| | |
|---|---|
| Leucine | 5 to 12 g/l |
| Isoleucine | 3 to 10 g/l |
| Valine | 5 to 10 g/l |
| Tryptophan | 1.0 to 3 g/l |
| Phenylalanine | 1.5 to 7 g/l |
| Lysine | 2 to 7 g/l |
| Methionine | 1.5 to 5 g/l |
| Threonine | 3.0 to 7 g/l. |

16. Use according to any one of Claims 11 to 15, **characterized in that** the composition is intended to be administered to man.

17. Use according to any one of Claims 11 to 15, **characterized in that** the composition is intended to be administered to animals.

## Patentansprüche

1. Zubereitung auf der Grundlage von Aminosäuren zur Verabreichung auf oralem, enteralem oder parenteralem Wege, **dadurch gekennzeichnet, daß** sie in einem biologisch und ernährungsmäßig annehmbaren Medium mindestens Cystein in freier Form oder in Form eines Vorläufers, eines Vorwirkstoffs, eines Proteins, eines Peptidhydrolysats in einem Anteil von gleich oder größer 3 %, bezogen auf die Gesamtheit der in der Zubereitung vorhandenen Aminosäuren; mindestens Threonin in Anteilen von gleich oder größer 5 % und mindestens Serin in Anteilen gleich oder größer 12 % und mindestens Asparaginsäure oder Asparagin in Anteilen von gleich oder größer 10 %, wobei diese Anteile auf die Menge der in der Zubereitung vorhandenen Aminosäuren bezogen bestimmt sind; und osidische und lipidische Kalorienquellen enthält.

2. Zubereitung nach Anspruch 1 in Form einer vollständigen Ernährungszubereitung bestimmt zur Verabreichung auf parenteralem Wege, welches Präparat neben den Aminosäuren oder deren Derivaten (Peptide) und den osidischen und lipidischen Kalorienquellen Elektrolyte, Spurenelemente und/oder Vitamine enthält.

3. Zubereitung nach Anspruch 1, **dadurch gekennzeichnet, daß** die Zubereitung in Form einer oral oder enteral zu verabreichenden Ernährungszubereitung vorliegt.

4. Zubereitung in Form einer Ernährungszubereitung, die für die orale oder enterale Verabreichung bestimmt ist, **dadurch gekennzeichnet, daß** sie in einem biologisch und näherstoffmäßig annehmbaren Medium mindestens Cystein in freier Form oder in Form seiner Salze, von Protein oder Peptidhydrolysat in Anteilen von gleich oder größer 3 %, bezogen auf die Gesamtheit der in der Zubereitung vorhandenen Aminosäuren, enthält und zusätzlich osidische und/oder lipidische Kalorienquellen, Elektrolyte, Spurenelemente und/oder Vitamine und mindestens Threonin in Anteilen von gleich oder größer 5 % und mindestens Serin in Anteilen von gleich oder größer 12 % und mindestens Asparaginsäure oder Asparagin in Anteilen von gleich oder größer 10 %, wobei diese Anteile auf die Gesamtheit der in der Zubereitung vorhandenen Aminosäuren bezogen bestimmt sind, umfaßt.

5. Zubereitung nach einem der Ansprüche 1 bis 4, **dadurch gekennzeichnet, daß** der Stickstoffgehalt des Cysteins gleich oder größer 2,15 % ist, bezogen auf die Gesamtmenge des Stickstoffs.

6. Zubereitung nach einem der Ansprüche 1 bis 5, **dadurch gekennzeichnet, daß** sie mindestens die 8 essentiellen Aminosäuren Leucin, Isoleucin, Valin, Tryptophan, Phenylalanin, Lysin, Methionin und Threonin enthält.

7. Zubereitung nach einem der Ansprüche 1 bis 6, **dadurch gekennzeichnet, daß** die Zubereitung zusätzlich Glycin und/oder Arginin enthält.

8. Zubereitung nach einem der Ansprüche 1 bis 7, **dadurch gekennzeichnet, daß** sie zusätzlich Taurin und/oder Glutamin enthält.

9. Zubereitung nach einem der Ansprüche 1 bis 8, **dadurch gekennzeichnet, daß** sie pro Liter Lösung der Aminosäuren:
| | |
|---|---|
| Leucin | 5 bis 12 g/l |
| Isoleucin | 3 bis 10 g/l |
| Valin | 5 bis 10 g/l |
| Tryptophan | 1,0 bis 3 g/l |
| Phenylalanin | 1,5 bis 7 g/l |
| Lysin | 2 bis 7 g/l |
| Methionin | 1,5 bis 5 g/l |
| Threonin | 3,0 bis 7 g/l |
enthält.

10. Zubereitung nach einem der Ansprüche 1 bis 9, **dadurch gekennzeichnet, daß** sie in Form eines zum Zeitpunkt der Verabreichung rehydratisierbaren Pulvers vorliegt.

11. Verwendung, für die Herstellung eines Arzneimittels zur Vorbeugung oder Verringerung von Gewebeschäden, die durch Stoffwechselfunktionsstörungen verursacht sind, einer Zubereitung auf der Grundlage von Aminosäuren, die bestimmt ist zur Verabreichung auf oralem, enteralem oder parenteralem Wege und in einem biologisch und nährstoffmäßig annehmbaren Medium mindestens Cystein in freier Form oder in Form eines Vorläufers, eines Vorwirkstoffs, eines Proteins, eines Peptidhydrolysats in einem Anteil von gleich oder größer 3 %, bezogen auf die Gesamtheit der in der Zubereitung vorhandenen Aminosäuren und mindestens Threonin in Anteilen von gleich oder größer 5 % und mindestens Serin in Anteilen von gleich oder größer 12 % und mindestens Asparaginsäure oder Asparagin in Anteilen von gleich oder größer 10 %, wobei diese Anteile auf die Gesamtheit der in der Zubereitung vorhandenen Aminosäuren bezogen bestimmt sind, enthält.

12. Verwendung nach Anspruch 11, **dadurch gekennzeichnet, daß** die Stoffwechselfunktionsstörungen eine Folge von Infektionen oder Reizungen, die eine Entzündungsreaktion verursachen, bedingt sind.

13. Verwendung nach einem der Ansprüche 1 bis 12, **dadurch gekennzeichnet, daß** die Zubereitung mindestens die 8 essentiellen Aminosäuren Leucin, Isoleucin, Valin, Tryptophan, Phenylalanin, Lysin, Methionin und Threonin enthält.

14. Verwendung nach einem der Ansprüche 1 bis 13 **dadurch gekennzeichnet, daß** die Zubereitung zusätzlich Glycin und/oder Arginin und /oder Taurin und/oder Glutamin enthält.

15. Verwendung nach einem der Ansprüche 1 bis 14, **dadurch gekennzeichnet, daß** die Zubereitung pro Liter der Lösung der Aminosäuren:
| | |
|---|---|
| Leucin | 5 bis 12 g/l |
| Isoleucin | 3 bis 10 g/l |
| Valin | 5 bis 10 g/l |
| Tryptophan | 1,0 bis 3 g/l |
| Phenylalanin | 1,5 bis 7 g/l |
| Lysin | 2 bis 7 g/l |
| Methionin | 1,5 bis 5 g/l |
| Threonin | 3,0 bis 7 g/l |
enthält.

16. Verwendung nach einem der Ansprüche 11 bis 15, **dadurch gekennzeichnet, daß** die Zubereitung an den Menschen verabreichbar ist.

17. Verwendung nach einem der Ansprüche 11 bis 15, **dadurch gekennzeichnet, daß** die Zubereitung an Tiere verabreichbar ist.
